# EUROPÄISCHE PATENTANMELDUNG

(11) **EP 4 129 267 A1**
(43) Veröffentlichungstag der Anmeldung: **08.02.2023**
(21) Anmeldenummer: 22174363.6
(22) Anmeldetag: 19.05.2022
(51) Int. Cl.: A61K 8/73, A61K 8/34, A61K 8/41, A61K 8/06, A61Q 5/06

(54) **HAAR-STYLING-EMULSION ENTHALTEND EIN NATÜRLICHES STYLING-POLYMER**

(30) Priorität: 23.06.2021 DE 102021206452
(71) Anmelder: Beiersdorf AG, 20245 Hamburg (DE)
(72) Erfinder: Budnick, Nina, Hamburg 22147 (DE); Behrendt, Pia, Hamburg 22455 (DE); Nemnich, Julia, 22589 Hamburg (DE)
(74) Vertreter: Beiersdorf AG

(57) **Zusammenfassung**

Haar-Styling-Emulsion enthaltend wenigstens ein natürliches Styling-Polymer.

## Beschreibung

Die vorliegende Erfindung liegt auf dem kosmetischen Gebiet von Haarzubereitungen, und zwar auf dem Gebiet von Haar-Styling-Zubereitungen, die dadurch gekennzeichnet sind, dass wenigstens ein natürliches Styling-Polymer enthalten ist.

Haare sind ein wichtiger Teil des menschlichen Körpers. Der ganze menschliche Körper mit Ausnahme der Lippen, der Handinnenflächen und der Fußsohlen ist behaart, zum Großteil allerdings mit kaum sichtbaren Wollhärchen. Wegen der vielen Nervenenden an der Haarwurzel reagieren Haare empfindlich auf äußere Einflüsse wie Wind oder Berührung und sind daher ein nicht zu unterschätzender Bestandteil des Tastsinns. Die wichtigste Funktion des menschlichen Kopfhaares dürfte allerdings heute darin bestehen, das Aussehen des Menschen in charakteristischer Weise mitzugestalten. Ähnlich wie die Haut erfüllt das Haar eine soziale Funktion, da es über sein Erscheinungsbild erheblich zu zwischenmenschlichen Beziehungen und zum Selbstwertgefühl des Individuums beiträgt.

Das Haar besteht aus einem Haarschaft, der frei aus der Haut herausragt. Der Haarschaft ist keratinisiert (tot) und ist der sichtbare Teil des Haares. Die nicht-sichtbare Haarwurzel ist der lebende Teil und in der Haut lokalisiert. In der Haarwurzel wird das Haar gebildet. Der Haarschaft ist aus drei Schichten aufgebaut: einem zentralen Teil, dem so genannten Haarmark (Medulla), das beim Menschen allerdings zurückgebildet ist und oft gänzlich fehlt, dem Mark (Cortex) und der äußeren, bis zu zehn Lagen starken, Schuppenschicht (Cuticula), die das ganze Haar umhüllt.

Das menschliche Haar ist, sofern keine krankhaften Veränderungen vorliegen, in seinem frisch nachgewachsenen Zustand praktisch nicht zu verbessern. Der in der Nähe der Kopfhaut befindliche Teil eines Haares weist dementsprechend eine nahezu geschlossene Schuppenschicht auf.

Natürliches Haar hat in vielen Fällen wenig eigenes Volumen und bei langem Haar hängt es oftmals recht schlaff und gerade vom Kopf herab. Viele Menschen wünschen sich aber Haare, die mehr Volumen aufweisen und sich auf gefällige Art formen (stylen) lassen. Dies kann grundsätzlich auf zweierlei Weise erzielt werden, zum einen gibt es permanente Haarverformungsverfahren, beispielsweise die Dauerwelle und nicht permanente Verfahren, die die Frisur des Haares nur für eine begrenzte Zeit formen und fixieren. Um eine nicht-permanente Frisurgestaltung zu erreichen, werden mehrere Produktformen zur Verfügung gestellt, die einzeln oder in Kombination miteinander verwendet werden können. Zu nennen seien hier beispielsweise, Festiger, Schaumfestiger, Haarsprays, Haarwachse, Haargele und andere mehr.

Beim Verbraucher sehr beliebt sind Haar-Styling-Emulsionen, die in der Regel auf das nasse Haar aufgetragen werden. Anschließend wird das Haar zu einer Frisur geformt (gestylt), meist mithilfe eines Föns. Die Haar-Styling-Emulsionen zeichnen sich dadurch aus, dass sie Polymere enthalten, die eine Fixierung der Frisur bewirken und so zum Frisurerhalt geeignet sind. Zumeist werden derartige Produkte nach jeder Haarwäsche angewandt.

Im Stand der Technik gibt es bereits Offenbarungen zu Haar-Styling-Emulsionen, die als Schaum vorliegen.

Als Beispiel sei das Dokument DE 102018221615 A1 genannt, eine Styling-Emulsion, die neben einem Styling-Effekt auch eine pflegende Wirkung aufweist. Diese Emulsionen sind dadurch charakterisiert, dass sie synthetische Polymere enthalten, die für die festigende und Film-bildende Wirkung wichtig sind, nämlich Polymere, die wenigstens ein Vinyl-Pyrrolidon-Monomer enthalten und kationische Polymere wie beispielsweise Polyquaternium-37.

Aus Verbraucherumfragen wird zunehmend deutlich, dass die Verbraucherinnen immer mehr wert darauflegen, dass natürliche Substanzen oder Substanzen, die auf natürlichen Verbindungen basieren, in die kosmetischen Produkte eingearbeitet werden. Dies gilt auch für Styling-Zubereitungen allgemein und somit auch für Styling-Zubereitungen auf Emulsions-Basis.

Synthetische Polymere sind, wie der Name aussagt, synthetische Verbindungen, für die es in der Regel keine natürlichen Abbaumechanismen gibt. Die Folge ist, dass derartige Verbindungen nicht gut, sehr schlecht oder fast gar nicht biologisch abbaubar sind.

Wünschenswert ist jedoch der Einsatz von Polymeren in Haar-Styling-Emulsionen und allgemein in kosmetischen Zubereitungen, die eine gute biologische Abbaubarkeit aufweisen. Dies ist für Polymere, die natürlichen Ursprungs sind in der Regel gegeben oder deutlich besser gegeben.

Der Begriff "natürlich" im Zusammenhang mit natürlichen Inhaltsstoffen für Kosmetika ist in der ISO 16128 festgelegt. Gemäß dieser Richtlinie kann man natürliche, kosmetische Inhaltsstoffe zusammenfassend als Stoffe beschreiben, die von Pflanzen, Tieren, Mineralien oder Mikroorganismen erhältlich sind, eingeschlossen sind auch solche Stoffe, die von den genannten Quellen durch physikalische Prozesse, Fermentationsprozesse (nur solche Fermentationsprozesse, die auch in der Natur ablaufen und die zu Produkten führen, die auch in der Natur entstehen) und andere Herstellungsmethoden ohne beabsichtigte chemische Modifikation erhalten werden. Die Mikroorganismen dürfen nur solche sein, die nicht gentechnisch modifiziert wurden.

Im Sinne der vorliegenden Erfindung sind Substanzen natürlichen Ursprungs, insbesondere Polymere natürlichen Ursprungs, Verbindungen, die von natürlichen Substanzen/Polymeren durch Modifizierungen abgeleitet sind. Damit sind auch chemische Modifizierungen umfasst.

Der Begriff der biologischen Abbaubarkeit beschreibt den Prozess des Abbaus von organischen Verbindungen durch Lebewesen, insbesondere Saprobionten. Im Idealfall entstehen anorganische Stoffe, wie beispielsweise CO₂, O₂ und Ammoniak; Verbindungen, die von Pflanzen und Mikroorganismen wieder für den Aufbau von organischen Verbindungen genutzt werden.

Organische, chemische Verbindungen, die leicht biologisch abbaubar sind, sind nach OECD 301, für kosmetische Zusammensetzungen meist nach OECD 301 B klassifiziert. Diese Substanzen oder Zusammensetzungen können rasch und vollständig abgebaut werden.

Organische, chemische Verbindungen, die nach OECD 302 klassifiziert sind, sind zwar eingeschränkt, jedoch grundsätzlich biologisch abbaubar.

Die Aufgabe der vorliegenden Erfindung war es, eine Haar-Styling-Emulsion zur Verfügung zu stellen, die Polymere enthält, die natürlichen Ursprungs sind. Die Menge an synthetischen Polymeren, die einen Beitrag zu den festigenden Eigenschaften der Haar-Styling-Emulsion leisten, sollte verringert werden oder es sollte komplett auf derartige Polymere verzichtet werden. Die erfindungsgemäße Haar-Styling-Emulsion sollte jedoch in den Anwendungseigenschaften, Festigung und Frisurerhalt, mit herkömmlichen Haar-Styling-Emulsionen vergleichbar sein.

Gelöst wurden die vorstehenden Aufgaben durch eine Haar-Styling-Emulsion, die wenigstens ein natürliches, wasserlösliches, lineares Polymer mit festigender/Film-bildender Wirkung, insbesondere Pullulan, enthält.

Im StdT sind bereits Zusammensetzungen beschrieben, die zur Festigung und Formung von Haaren eingesetzt werden können und keine synthetischen Polymere enthalten. Als Beispiel sei das Dokument WO 2018/160384 A1 genannt.

Ebenso sind im StdT Dokumente veröffentlicht, die die Verwendung von Pullulan, offenbaren.

Das Dokument US 2020/0000701 A1 beschreibt Haar-Stylingzusammensetzungen, die Pullulan und wenigstens eine Cellulose-Ether-Verbindung enthalten.

Im Dokument WO 2020/127069 A1 werden Haarzusammensetzungen beschrieben, die zum Stylen der Haare und zur Definition von Locken eingesetzt werden können. Diese Zusammensetzungen enthalten u.a. Polysaccharide. Ein bevorzugtes Polysaccharid ist Pullulan.

Die Aufgabe der vorliegenden Erfindung war es also, eine Haar-Styling-Emulsion zur Verfügung zu stellen, die wenigstens ein Polymer enthält, das natürlichen Ursprungs ist. Die Menge an synthetischen Polymeren, die einen Beitrag zu den festigenden/Film-bildenden Eigenschaften der Haar-Styling-Emulsion leisten, sollte verringert werden oder es sollte komplett auf derartige Polymere verzichtet werden. Die erfindungsgemäße Haar-Styling-Emulsion sollte jedoch in den Anwendungseigenschaften, Festigung und Frisurerhalt, mit herkömmlichen Haar-Styling-Emulsionen vergleichbar sein.

Gelöst werden die vorstehenden Aufgaben durch eine Haar-Styling-Emulsion enthaltend
- Pullulan in einer Menge von 0,1 bis 10,0 Gew.-%,
- wenigstens zwei Moisturizer, insbesondere vorliegend mit einem Gesamtgewicht von wenigstens 5 Gew.-%, bezogen auf das Gesamtgewicht der Emulsion,
- wenigstens ein Fettalkohol, insbesondere vorliegend mit einem Gesamtgewicht von wenigstens 3,0 Gew.-%, bezogen auf das Gesamtgewicht der Emulsion,
- optional wenigstens einen Emulgator und/oder wenigstens ein kationisches Tensid,
- optional wenigstens ein Verdicker natürlichen Ursprungs, mit der Maßgabe, dass der genannte Verdicker nicht Pullulan ist, insbesondere wenigstens eine modifizierte Stärkeverbindung.

Ebenso Gegenstand der Erfindung ist ein Haar-Styling-Emulsionsprodukt bestehend aus einer Haar-Styling-Emulsion und einer Verpackungseinheit, insbesondere ein Tiegel oder eine Tube.

Die erfindungsgemäße Haar-Styling-Emulsion ist eine kosmetische Zubereitung, die vorteilhaft auf das menschliche Kopfhaar aufgetragen wird und nicht wieder ausgespült wird (leave-on). Weiter vorteilhaft liegt die erfindungsgemäße Zubereitung in Form einer O/W-Emulsion. Die erfindungsgemäße Zubereitung ist eine wässrige Zubereitung.

Der Wassergehalt der erfindungsgemäßen Emulsion beträgt vorteilhaft wenigstens 50 Gew.-%, bevorzugt wenigstens 55 Gew.-%, bezogen auf das Gesamtgewicht der Emulsion. Der Wassergehalt der erfindungsgemäßen Emulsion beträgt vorteilhaft höchstens 97 Gew.-%, bezogen auf das Gesamtgewicht der Emulsion.

Das natürliche, wasserlösliche, lineare Polymer mit festigender/Film-bildender Wirkung ist Pullulan. Pullulan ist ein α-1,6 verknüpftes Polysaccharid aus Maltotriose-Einheiten, das aus dem Pilz *Aureobasidium pullulans* gewonnen werden kann. Die Glucose-Einheiten der Maltotriose sind α-1,4-glycosidisch miteinander verknüpft. Pullulan ist gut in Wasser löslich.

Pullulan kann beispielsweise unter dem Handelsnamen Pullulan von der Firma Hayashibara Co., LTD. bezogen werden.

In der erfindungsgemäßen Emulsion ist Pullulan mit einem Gehalt von 0,2 bis 8,0 Gew.-%, bevorzugt von 0,5 bis 6,0 Gew.-% enthalten, bezogen auf das Gesamtgewicht der Emulsion.

Die erfindungsgemäße Haar-Styling-Emulsion enthält wenigstens zwei Moisturizer, insbesondere vorliegend mit einem Gesamtgewicht von wenigstens 5 Gew.-%, bezogen auf das Gesamtgewicht der Emulsion. Als Moisturizer werden Stoffe oder Stoffgemische bezeichnet, welche kosmetischen Zubereitungen die Eigenschaft verleihen, nach dem Auftragen bzw. Verteilen auf der Haaroberfläche die Feuchtigkeitsabgabe zu reduzieren und/oder die Hydratation der Haaroberfläche bzw. der tiefer liegenden Schichten positiv zu beeinflussen. Vorteilhafte Moisturizer im Sinne der vorliegenden Erfindung sind beispielsweise Glycerin, Sorbitol, Butylenglykol, Propylenglykol, Milchsäure und/oder Lactate, insbesondere Natriumlactat, Biosaccaride Gum-1, Glycine Soja, Pyrrolidoncarbonsäure und Harnstoff. Bevorzugt werden die Moisturizer ausgewählt aus der Gruppe Glycerin, Sorbitol, Butylenglykol, Propylenglykol, Milchsäure und/oder Lactate, insbesondere Natriumlactate. Es hat sich als vorteilhaft erwiesen, wenn zwei verschiedene Moisturizer in der erfindungsgemäßen Emulsion enthalten sind. Die Feuchtigkeit des Haares ist dadurch weiter verbessert und ebenso vorteilhaft ist die Sensorik der Emulsion, die insgesamt als sehr angenehm und gerade richtig empfunden wird; also nicht zu fest oder dünnflüssig und angenehm im Gefühl auf der Haut (Hände, die die Emulsion auftragen) und den Haaren. Deshalb sind in der erfindungsgemäßen Emulsion wenigstens zwei Moisturizer enthalten, wobei bevorzugt wenigstens einer der Moisturizer aus der Gruppe der bevorzugten Verbindungen ausgewählt wird. Weiter bevorzugt sind wenigstens zwei der Moisturizer aus der ausgewählten Gruppe ausgewählt. Am meisten bevorzugt sind in der erfindungsgemäßen Emulsion Glycerin und Sorbitol enthalten.

Die wenigstens zwei Moisturizer liegen in der erfindungsgemäßen Emulsion bevorzugt mit einem Gesamtgehalt von 5,5 bis 30,0 Gew. %, besonders bevorzugt 6,0 bis 27,0 Gew. %, insbesondere bevorzugt 7,5 bis 25,0 Gew. %, bezogen auf das Gesamtgewicht der Emulsion, vor.

In der erfindungsgemäßen Emulsion ist wenigstens ein Fettalkohol enthalten, insbesondere vorliegend mit einem Gesamtgewicht von wenigstens 3,0 Gew.-%, bezogen auf das Gesamtgewicht der Emulsion.

Fettalkohole sind aliphatische, langkettige, einwertige, meist primäre Alkohole. Der Begriff Fettalkohol wird in der Regel für Fettalkohole mit einer Kettenlänge von 6 bis 22 Kohlenstoffatomen verwendet. Die Kohlenstoffkette der Fettalkohole kann eine oder mehr Doppelbindungen aufweisen und/oder unverzweigt oder verzweigt sein.

Es ist bevorzugt, wenn in der erfindungsgemäßen Emulsion der wenigstens eine Fettalkohol unverzweigt und/oder gesättigt ist, weiter bevorzugt unverzweigt und gesättigt.

Die Fettalkohole werden bevorzugt aus der Gruppe Myristylalkohol, Cetylalkohol, Stearylalkohol und/oder Cetearylalkohol ausgewählt, besonders bevorzugt sind Myristylalkohol und/oder Cetylalkohol.

In der erfindungsgemäßen Emulsion ist der wenigstens eine Fettalkohol bevorzugt mit einem Gesamtgehalt von 4,0 bis 15,0 Gew. %, besonders bevorzugt von 4,0 bis 10,0 Gew.-% enthalten, bezogen auf das Gesamtgewicht der Emulsion.

In vorteilhaften Ausführungsformen der vorliegenden Erfindung ist wenigstens ein Emulgator, bevorzugt wenigstens zwei Emulgatoren enthalten.

Emulsionen benötigen zu ihrer Bildung und zur Stabilisierung im Allgemeinen einen oder mehrere Emulgatoren, gegebenenfalls auch Verdicker und/oder Konsistenzgeber, um über einen kosmetisch akzeptablen Zeitraum stabil zu sein.

Emulgatoren bewirken, dass die zwei nicht miteinander mischbaren Flüssigkeiten (zum Beispiel Öl in Wasser) sich zu einer Emulsion vermengen lassen. Aufgrund des amphiphilen Charakters dringen sie mit ihrem fettlöslichen Teil in das Öl ein. Durch den hydrophilen Teil können die durch Rühren oder Homogenisieren entstandenen Öltröpfchen in der wässrigen Umgebung dispergiert werden. Emulgatoren haben primär keinen waschaktiven, tensidischen Charakter.

Emulgatoren setzen die Grenzflächenspannung zwischen den beiden Phasen herab und erreichen neben der Verringerung der Grenzflächenarbeit auch eine Stabilisierung der gebildeten Emulsion. Sie stabilisieren die gebildete Emulsion durch Grenzflächenfilme sowie durch Ausbildung sterischer oder elektrischer Barrieren, wodurch das Zusammenfließen (Koaleszenz) der emulgierten Teilchen verhindert wird.

Damit Verbindungen als Emulgatoren wirksam sein können, müssen sie eine bestimmte Molekülstruktur aufweisen. Strukturelles Kennzeichen solcher Verbindungen ist ihr amphiphiler Molekülaufbau. Das Molekül einer solchen Verbindung besitzt wenigstens eine Gruppe mit Affinität zu Substanzen starker Polarität (polare Gruppe) und wenigstens eine Gruppe mit Affinität zu unpolaren Substanzen (apolare Gruppe).

Vorteilhafte Emulgatoren können aus der Gruppe Trilaureth-4 Phosphate (beispielsweise erhältlich unter Handelsbezeichnung Hostaphat KL 340 D MB bei der Firma Clariant SE), Ceteareth-20 (beispielsweise erhältlich unter Handelsbezeichnung Eumulgin B2 von der Firma BASF), Sodium Stearoyl Glutamate (beispielsweise erhältlich unter Handelsbezeichnung Eumulgin SG von der Firma BASF), Glyceryl Stearate (beispielsweise erhältlich unter Handelsbezeichnung Tegin M Pellets MB von der Firma Evonik), Sodium Cetearyl Sulfate (beispielsweise erhältlich unter Handelsbezeichnung Lanette E granules von der Firma BASF), Glyceryl Stearate SE (beispielsweise erhältlich unter Handelsbezeichnung Tegin VS MB von der Firma Evonik oder unter Handelsbezeichnung Cutina GMS-SE von der Firma BASF) und/oder Sorbitanmonostearat (beispielsweise erhältlich unter Handelsbezeichnung SP SPAN 60 MBAL von der Firma Croda GmbH).

In der erfindungsgemäßen Emulsion sind bevorzugt die Emulgatoren Trilaureth-4 Phosphat und/oder Sorbinatstearat enthalten

Wenn in der erfindungsgemäßen Haar-Styling-Emulsion wenigstens ein Emulgator enthalten ist, so liegt dieser wenigstens eine Emulgator mit einem Gesamtgehalt von 0,5 bis 4,0 Gew. %, bevorzugt 1,0 bis 3,0 Gew. % vor, bezogen auf das Gesamtgewicht der Emulsion.

In einer ebenso vorteilhaften Ausführungsform der vorliegenden Erfindung ist kein Emulgator im "klassischen Sinne" enthalten, wohl aber wenigstens ein kationisches Tensid, das als Emulgator wirkt und die Emulsion stabilisiert. Darüber hinaus leisten auch der/die enthaltene(n) Fettalkohol(e) einen Beitrag, um die Emulsion zu stabilisieren.

Dieses kationische Tensid wird bevorzugt aus der Gruppe der Amidoamine. Diese Verbindungen können erhalten werden durch die Reaktion von Fettsäuren mit Diaminen und nachfolgender Quaternierung der freien Aminfunktion. Es ist weiter bevorzugt, wenn das kationische Tensid ausgewählt wird aus der Gruppe Stearylamidopropyltrimmonium Chloride, Ricinoleamidopropyltrimonnium Chloride und/oder Palmitamidopropyltrimonium Chloride; am meisten bevorzugt ist es, wenn das kationische Tensid Palmitamidopropyltrimonium Chlorid ist. Palmitamidopropyltrimonium Chlorid kann als Varisoft PATC bei der Firma Evonik Industries bezogen werden.

Das wenigstens eine kationische Tensid ist in der erfindungsgemäßen Zubereitung mit einem Gehalt von 0,1 bis 4 Gew.-%, bevorzugt 0.2 bis 2 Gew.-% enthalten, bezogen auf das Gesamtgewicht der Zubereitung und bezogen auf den Aktivgehalt.

Die erfindungsgemäße Emulsion kann optional wenigstens einen Verdicker natürlichen Ursprungs enthalten. Bei den genannten Verdickern handelt es sich bevorzugt um Polymere ausgewählt aus der Gruppe der Stärkeverbindungen, die modifiziert sind. Die Stärkeverbindungen können aus verschiedenen Quellen stammen, beispielsweise Reisstärke, Maisstärke oder Kartoffelstärke. Die Gruppe der modifizierten Stärkeverbindungen hat auch Film-bildende Eigenschaften. Im Zusammenhang mit der vorliegenden Erfindung sind jedoch die verdickenden Eigenschaften relevant und tragen zur Erfindung bei. Die erfindungsgemäßen Stärkeverbindungen sind nichtionische Biopolymere. Stärke ist ein Speicherkohlenhydrat der Pflanzen, das aus zwei Molekültypen besteht, Amylose und Amylopektin. Kennzeichnend für Amylose sind lineare helikale Ketten aus α-1,4-glykosidisch verknüpften Glukosemolekülen, während Amylopektin eine verzweigte Struktur aufweist. Diese Verzweigung wird durch zusätzliche α 1,6 glykosidisch verknüpfte Glukosemoleküle erreicht.

Erfindungsgemäß weiter bevorzugt ist der Einsatz wenigstens einer modifizierten Maisstärkeverbindung. Die Begriffe Maisstärkeverbindung und Maisstärke werden im Zusammenhang mit dieser Erfindung synonym verwendet.

Modifizierte Maisstärke kann beispielsweise als Hydroxypropylstärke vorliegen, die INCI-Bezeichnung lautet Corn Starch Modified. Eine derartige Verbindung ist beispielsweise bei der Firma Nouryon unter der Handelsbezeichnung Amaze erhältlich. Darüber hinaus kann Hydroxypropylstärke auch unter der Handelsbezeichnung Agenajel 20.383 von der Firma Agrana bezoegen werden. Weiterhin kann die Stärke auch als hydrolysierte Stärke vorliegen, dabei wird die Stärke meist durch eine Säurebehandlung, aber auch enzymatisch, zu Zwischenprodukten abgebaut. Derartige Produkte haben die INCI-Bezeichnung Hydrolyzed Corn Starch (erhältlich beispielsweise als MaizeCare Style Polymer bei der Firma Dow) oder Maltodextrin (erhältlich beispielsweise als Agenanova 30.326 von der Firma Agrana). Darüber hinaus kann die modifizierte Maisstärke auch als Distärkephosphat vorliegen, erhältlich beispielsweise als Agenajel 20.306 von der Firma Agrana. Diese Verbindung hat die INCI-Bezeichnung Distarch Phosphate. Eine Kombination aus hydroxypropylierter Stärke mit phosphatierter Stärke hat die INCI-Bezeichnung Hydroxyproyl Starch Phosphate und ist beispielsweise unter Bezeichnung Agenajel 20.383 Bei der Firma Agrana erhältlich.

Modifizierte Kartoffelstärke kann beispielsweise als hydroxypropylierte Stärke vorliegen; diese Verbindung hat die INCI-Bezeichnung Hydroxypropyl Starch und ist beispielsweise erhältlich bei der Firma Agrana unter der Handelsbezeichnung Amitrolit 8850.

Ebenso geeignet und vorteilhaft ist der Einsatz von Kartoffelstärke, die nicht modifiziert ist; eine derartige Kartoffelstärke ist beispielsweise erhältlich mit der INCI Bezeichnung Solanum Tuberosum Starch (Handelsname Stärkina Natural, Firma Agrana).

Modifizierte Reisstärke kann beispielsweise als Distärkephosphat vorliegen; diese verbindung hat die INCI-Bezeichnung Distarch Phosphate und ist beispielsweise mit der Handelsbezeichnung Rice PO4 Natural bei der Firma Agrana erhältlich.

Bevorzugt ist der Einsatz einer hydroxypropylierten Maisstärke mit der INCI-Bezeichnung Corn Starch Modified.

Wenn in der erfindungsgemäßen Haar-Styling-Emulsion wenigstens ein Verdicker natürlichen Ursprungs, insbesondere ausgewählt aus der Gruppe der modifizierten Stärkeverbindungen, enthalten ist, so liegt dieser Verdicker vorteilhaft mit einem Gesamtgehalt von 0,1 bis 8,0 Gew. %, bevorzugt von 0,5 bis 5,0 Gew. % vor, bezogen auf das Gesamtgewicht der Emulsion.

In der erfindungsgemäßen Emulsion können zusätzlich zu dem wenigstens einen Fettalkohol weitere Öl- und/oder Wachskomponenten enthalten sein.

Die Öl- und/oder Wachskomponenten können vorteilhaft polare Öle umfassen. Die Gruppe der polaren Öle umfasst insbesondere vorteilhaft die Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z. B. Cocoglycerid, Olivenöl, Sonnenblumenöl, Sojaöl, Erdnussöl, Rapsöl, Mandelöl, Palmöl, Kokosöl, Rizinusöl, Weizenkeimöl, Traubenkernöl, Distelöl, Nachtkerzenöl, Macadamianssöl und andere mehr.

Die Öl- und/oder Wachskomponenten können ebenso vorteilhaft natürliche Wachse tierischen und pflanzlichen Ursprungs umfassen, wie beispielsweise Bienenwachs und andere Insektenwachse sowie Beerenwachs, Sheabutter und/oder Lanolin (Wollwachs), wobei Sheabutter besonders vorteilhaft ist.

Die Öl- und/oder Wachskomponenten können auch vorteilhaft Ester aus gesättigten und/oder ungesättigten, verzweigten und/oder unverzweigten Alkancarbonsäuren einer Kettenlänge von 3 bis 30 C Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen umfassen. Solche Esteröle können dann besonders vorteilhaft gewählt werden aus der Gruppe Octylpalmitat, Octylcocoat, Octylisostearat, Octyldodeceylmyristat, Octyldodekanol, Cetearylisononanoat, Isopropylmyristat, Isopropylpalmitat, Isopropylstearat, Isopropyloleat, n-Butylstearat, n-Hexyllaurat, n Decyloleat, Isooctylstearat, Isononylstearat, Isononylisononanoat, 2-Ethylhexylpalmitat, 2 Ethylhexyllaurat, 2-Hexyldecylstearat, 2-Octyldodecylpalmitat, Stearylheptanoat, Oleyloleat, Oleylerucat, Erucyl-oleat, Erucylerucat, Tridecylstearat, Tridecyltrimellitat, sowie synthetische, halbsynthetische und natürliche Gemische solcher Ester, wie z. B. Jojobaöl.

Auch besonders vorteilhaft enthält die erfindungsgemäße Zubereitung einen Gehalt an C12 15-Alkylbenzoat oder die Ölkomponente ist vorteilhaft C12-15-Alkylbenzoat.

Die Öl- und/oder Wachskomponenten können auch vorteilhaft Dialkylether und Dialkylcarbonate umfassen; vorteilhaft sind z. B. Dicaprylylether (Cetiol OE) und/oder Dicaprylylcarbonat, das beispielsweise unter der Handelsbezeichnung Cetiol CC bei der Fa. Cognis erhältlich ist.

Auch beliebige Abmischungen der genannten Öl- und Wachskomponenten sind vorteilhaft im Sinne der vorliegenden Erfindung einzusetzen.

Die Öl- und/oder Wachskomponenten können auch unpolare Öle, die gewählt werden aus der Gruppe der verzweigten und unverzweigten Kohlenwasserstoffe und -wachse, insbesondere Mineralöl, Vaseline (Petrolatum), Paraffinöl, Squalan, Polyolefine, hydrogenierte Polyisobutene und Isohexadecan, umfassen. Das Mineralöl ist vorteilhaft ein Gelböl, das beispielsweise unter der Handelsbezeichnung Rajell-UWP 12G10 von der Firma Raj Petro Sepcialities Pvt. Ltd. erhältlich ist.

Wenn in der erfindungsgemäßen Emulsion wenigstens eine zusätzliche Öl- und/oder Wachskomponente enthalten ist, so liegt die wenigstens eine zusätzliche Öl- und/oder Wachskomponente mit einem Gesamtgehalt von 0,1 bis 7,0 Gew. %, bevorzugt 0,5 bis 6,0 Gew. %, insbesondere bevorzugt 1,0 bis 5,0 Gew. %, bezogen auf das Gesamtgewicht der Emulsion, vor.

Wenngleich nicht bevorzugt, so kann die erfindungsgemäße Emulsion zusätzlich wenigstens ein nichtionisches fixierendes Polymer, welches Vinylpyrolidone-Monomere enthält, enthalten. Bevorzugt können diese Polymere ausgewählt werden aus der Gruppe der Homopolymere des Vinylpyrolidons und der Gruppe der Copolymere des Vinylpyrrolidons wie Copolymere aus Vinylpyrrolidon und Vinylacetat (VP/VA), Terpolymere aus, Polyvinylamide und deren Salze, Copolymere aus Vinylpyrrolidon und Dimethylaminoethylmethacrylat, Terpolymere aus Vinylcaprolactam, Vinylpyrrolidon und Dimethylaminomethacrylat. Besonders bevorzugt sind Copolymere aus Vinylpyrolidone und Vinylacetat, die unter der Handelsbezeichnung Luviskol VA 64 W bei der Firma BASF erhältlich sind. Ebenso besonders bevorzugt ist ein Homopolymer des Vinylpyrrolidons, das beispielsweise erhältlich ist unter Handelsbezeichnung Luviskol K 30 Powder von der Firma BASF.

Zu Vergleichszwecken enthalten die später beschriebenen Vergleichszubereitungen ein VP/VA Copolymer.

Wenn in der einer Haar-Styling-Emulsion wenigstens ein nichtionisches fixierendes Polymer, enthaltend Vinyl-Pyrrolidon-Monomere enthalten ist, so ist es mit einem Gehalt von 0,2 bis 8,0 Gew.-%, bevorzugt 0,5 bis 5,0 Gew.-% enthalten, bezogen auf das Gesamtgewicht der Emulsion und bezogen auf den Aktivgehalt.

Darüber hinaus können vorteilhaft weitere Komponenten in der erfindungsgemäßen Haar-Styling-Emulsion enthalten sein.

Weiterhin ist es vorteilhaft, wenn die Haar-Styling-Emulsion ein Parfüm enthält. Parfüme sind Mischungen aus Parfümrohstoffen. Das Parfüm liegt vorteilhaft mit einem Gehalt von 0.05 bis 1 Gew.-% vor, bezogen auf das Gesamtgewicht der Emulsion.

Ebenso ist es vorteilhaft, wenn die erfindungsgemäße Haar-Styling-Emulsion Konservierungsmittel enthält. Konservierungsmittel sind diejenigen konservierenden Substanzen, die gemäß Kosmetikverordnung für Deutschland und gemäß der Verordnung (EG) Nr. 1223/2009 über kosmetische Mittel für den Einsatz in kosmetischen Produkten für Europa zugelassen sind.

Als Konservierungsmittel können vorteilhaft Phenoxyethanol, Methyl-, Ethyl-, Propylparaben, Benzylalkohol, Sorbinsäure und/oder Salze der Sorbinsäure, beispielsweise Kalium Sorbat, Benzoesäure und/oder Salze der Benzoesäure, beispielsweise Natrium Benzoat, Salicylsäure und/oder Salze der Salicylsäure, beispielsweise Natrium Salicylat eingesetzt werden. Die genannten Konservierungsmittel können einzeln oder in Kombination eingesetzt werden.

Als Stabilisatoren sind im Sinne der vorliegenden Erfindung diejenigen Substanzen zu verstehen, die nicht in der Liste der zugelassenen Konservierungsstoffe (Kosmetikverordnung Anlage 6; Verordnung (EG) Nr. 1223/2009, Anhang V) aufgeführt sind, gleichwohl aber eine stabilisierende Wirkung haben und/oder im gemeinsamen Einsatz mit Konservierungsmitteln die Stabilität der Zubereitung 36 Monate fördern.

Als Stabilisatoren sind folgende Verbindungen vorteilhaft: Ethylhexylglycerin, 1,2-Hexanediol, Methylpropanediol, Caprylyl Glycol, Pentylene Glycol und/oder Hydroxyacetophenon.

Das wenigstens eine Konservierungsmittel und/oder der wenigstens eine Stabilisator liegen mit einem Gesamtgehalt von 0,01 bis 2,0 Gew. %, bevorzugt von 0,05 bis 1,5 Gew.-%, besonders bevorzugt von 0,1 bis 1,0 Gew. % in der erfindungsgemäßen Emulsion vor, bezogen auf das Gesamtgewicht der Emulsion.

Erfindungsgemäß vorteilhaft ist ebenfalls die Verwendung einer oder mehrerer Substanzen der Vitamine der B Gruppe und/oder des Vitamin B-Komplex, die dem erfindungsgemäßen Haargel zusätzlich zugesetzt werden können. Substanzen der Vitamin B Gruppe und/oder des Vitamin B-Komplex sind üblicherweise wasserlöslich und spielen insbesondere für den Zellmetabolismus bei Pflanzen und Tieren eine besondere Rolle.

Beispiele erfindungsgemäßer Substanzen der Vitamine der B Gruppe und/oder des Vitamin B-Komplex sind unter anderem Nicotinsäure (Vitamin B3), Nicotinsäureamid (Niacinamid), Panthenol (Provitamin B5); die Vitamine der B Gruppe und/oder des Vitamin B-Komplex sind vorteilhaft mit einem Gesamtgehalt von 0,01 bis 0,5 Gew. % enthalten, bezogen auf das Gesamtgewicht des Haargels.

Die erfindungsgemäße Emulsion kann frei von Silikonen sein.

Die Begriffe Silikon oder Silikonverbindung werden synonym verwendet. Silikonverbindungen zeichnen sich durch das Vorhandensein von Silicium- und Sauerstoffatomen aus, die miteinander verknüpft sind und eine Vielzahl verschiedener Verbindungen ausbilden können.

Im Sinne der vorliegenden Erfindung bedeutet der Begriff "frei von", dass weniger als 0,001 Gew. %, bevorzugt weniger als 0,0001 Gew. %, besonders bevorzugt 0 Gew. % der jeweiligen Substanz(en), bezogen auf das Gesamtgewicht der Emulsion, vorliegen.

Die erfindungsgemäße Emulsion ist in einem geeigneten Behälter enthalten, vorteilhaft in einer Tube oder einem Tiegel. Die Behälter sind vorteilhaft aus Kunststoff, insbesondere Polyethylenterephthalat (PET), Polypropylen (PP) oder Polyethylen (PE). Ebenso sind Behälter aus Glas oder Metall geeignet.

Um zu zeigen, dass die Merkmale des Gegenstandes der Erfindung, nämlich eine Haar-Styling-Emulsion enthaltend Pullulan in einer bestimmten Menge, wenigstens einen Emulgator, wenigstens zwei Moisturizer, wenigstens einen Fettalkohol und optional wenigstens einen natürlichen Verdicker, einen Beitrag zur Lösung der Aufgaben leisten, wurden verschiedene Haar-Styling-Emulsionen (Tabelle 1, Beispiele 1 bis 5 und Tabelle 2, Beispiele 4 bis 7) hergestellt. Eine Haar-Styling-Emulsion gemäß Beispiel 1 oder 4 enthielt als festigendes Polymer VP/VA (Beispiel 1) oder PVP (Beispiel 4); diese Haar-Styling-Emulsionen sind Vergleichszubereitungen und spiegeln den StdT wider. Bei der Bewertung dienen diese Beispiele als Standard und die Halteeigenschaften der erfindungsgemäßen Beispiele werden an diesem Standard gemessen, es wird also beurteilt, ob die Halteeigenschaften der erfindungsgemäßen Zubereitungen besser oder schlechter sind. Die Beispielformulierungen 2, 3, 5 bis 7 sind erfindungsgemäße Beispiele.

Je 0,4 g der zu untersuchenden Zubereitung wurden homogen auf 3D-Stylingtressen aufgetragen. Anschließend folgte ein Trocknungsschritt für 30 Minuten. Danach wurden die Tressen von 3 erfahrenen Mitarbeitern bewertet. Die Bewertung erfolgte in Stufen, hierbei wurde den Vergleichszubereitungen der Wert 0 zugeordnet. Eine bessere bzw. schlechtere Bewertung als diejenige der Vergleichszubereitung wurde folgendermaßen abgestuft:

| | |
|---|---|
| deutlich schlechter | --- |
| schlechter | -- |
| etwas schlechter | - |
| Standard | 0 |
| etwas besser | + |
| besser | ++ |
| Deutlich besser | +++ |

3D-Haartressen sind aus Euro-Natur-Haar aufgebaut, die auf ein Silikonblatt (4 x 24,5 cm) gestochen werden, wobei davon 3 x 10 cm bestochen und etwa 3 Haare pro Loch enthalten sind. Die Haarlänge beträgt etwa 3 cm und das Haargewicht etwa 1,2 bis 1,4 g.

Es zeigte sich, dass alle erfindungsgemäßen Haar-Styling-Emulsionen gleich gut (Beispiel 2) oder besser als der Standard bewertet wurden (Beispiele 3, 5 bis 7). Eine Zubereitung gemäß Beispiel 6, die neben Pullulan auch einen natürlichen Verdicker, nämlich hydrolysierte Maisstärke (Hydrolyzed Corn Starch), enthielt, wurde am besten bewertet und zeigte bessere Halteeigenschaften als die Vergleichsbeispiele. Somit konnte gezeigt werden, dass Haar-Styling-Emulsionen, die als festigendes/Film-Bildendes Polymer Pullulan enthalten, die vorgenannten Aufgaben zu lösen vermögen.

Gegenstand der vorliegenden Erfindung ist also auch die Verwendung von Pullulan in einer Menge von 0,1 bis 10,0 Gew. %, bezogen auf das Gesamtgewicht der Haar-Styling-Emulsion in Kombination mit wenigstens zwei Moisturizern, wenigstens einem Fettalkohol, wenigstens einem Emulgator und/oder wenigstens einem kationischen Tensid, und optional wenigstens einem Verdicker natürlichen Ursprungs, in einer Haar-Styling-Emulsion zur Festigung der Haare und zum Frisurerhalt.

Bevorzugt ist es, wenn der wenigstens eine Verdicker natürlichen Ursprungs hydrolysierte Maisstärke ist, bevorzugt vorliegend mit einem Gehalt von0,1 bis 8,0 Gew.-%, bezogen auf das Gesamtgewicht der Haar-Styling-Emulsion.

### Beispiele:

Die Beispielrezepturen sollen die Erfindung verdeutlichen, ohne sie einzuschränken. Die Gewichtsangaben beziehen sich auf Gewichtsprozentangaben und Aktivgehalte, sofern nicht anders angegeben.

| **INCI (Aktivgehalt)** | **1** | **2** | **3** | |
|---|---|---|---|---|
| Aqua | 84,6 | 84,6 | 83,6 | |
| VP/VA Copolymer | 1 | | | |
| Pullulan | | 1 | 2 | |
| Sorbitol | 1,6 | 1,6 | 1,6 | |
| Cetearyl Alcohol | 5 | 5 | 5 | |
| Palmitamidopropyl-trimonium Chloride | 0,5 | 0,5 | 0,5 | |
| Glycerin | 7 | 7 | 7 | |
| Parfum | 0,3 | 0,3 | 0,3 | |
| | | | | |
| Halteeigenschaften | o | o bis + | + | |

| **INCI (Aktivgehalt)** | **4** | **5** | **6** | **7** |
|---|---|---|---|---|
| Aqua | 65,4 | 65,4 | 64,2 | 65,9 |
| PVP | 1,5 | | | |
| Pullulan | | 1,5 | 1,5 | 1 |
| Hydrolyzed Corn Starch | | | 1,5 | |
| PEG-90 M | 0,3 | 0,3 | | 0,3 |
| Sorbitol | 9,5 | 9,5 | 9,5 | 9,5 |
| Myristyl Alcohol | 5 | 5 | 5 | 5 |
| Cetyl Alcohol | 5 | 5 | 5 | 5 |
| Sorbitan Stearate | 2 | 2 | 2 | 2 |
| Trilaureth-4 Phosphate | 1,8 | 1,8 | 1,8 | 1,8 |
| Glycerin | 9 | 9 | 9 | 9 |
| Parfum | 0,5 | 0,5 | 0,5 | 0,5 |
| | | | | |
| Halteeigenschaften | o | + | ++ | + |

## Patentansprüche

1. Haar-Styling-Emulsion enthaltend
- Pullulan in einer Menge von 0,1 bis 10,0 Gew.-%,
- wenigstens zwei Moisturizer, insbesondere vorliegend mit einem Gesamtgewicht von wenigstens 5 Gew.-%, bezogen auf das Gesamtgewicht der Emulsion,
- wenigstens ein Fettalkohol, insbesondere vorliegend mit einem Gesamtgewicht von wenigstens 3,0 Gew.-%, bezogen auf das Gesamtgewicht der Emulsion,
- wenigstens ein Emulgator und/oder ein kationisches Tensid,
- optional wenigstens einen natürlichen Verdicker, mit der Maßgabe, dass der oder die natürliche(n) Verdicker nicht Pullulan ist, insbesondere wenigstens eine modifizierte Stärkeverbindung.

2. Emulsion nach Anspruch 1, **dadurch gekennzeichnet, dass** die Haar-Styling-Emulsion auf das menschliche Kopfhaar aufgetragen wird und nicht wieder ausgespült wird (leave-on).

3. Emulsion nach wenigstens einem der vorstehenden Ansprüche, **dadurch gekennzeichnet, dass** der Wassergehalt wenigstens 50 Gew.-%, bevorzugt wenigstens 55 Gew.-%, insbesondere bevorzugt 60 Gew.-% und höchstens 97 Gew.-%, bezogen auf das Gesamtgewicht der Emulsion, beträgt.

4. Emulsion nach wenigstens einem der vorstehenden Ansprüche, **dadurch gekennzeichnet, dass** Pullulan mit einem Gehalt von 0,2 bis 8,0 Gew.-%, bevorzugt von 0,5 bis 6,0 Gew.-% enthalten ist, bezogen auf das Gesamtgewicht der Emulsion.

5. Emulsion nach wenigstens einem der vorstehenden Ansprüche, **dadurch gekennzeichnet, dass** der wenigstens eine Emulgator mit einem Gesamtgehalt von 0,5 bis 4,0 Gew.-%, bevorzugt 1,0 bis 3,0 Gew.-% enthalten, bezogen auf das Gesamtgewicht der Emulsion, vorliegt.

6. Emulsion nach wenigstens einem der vorstehenden Ansprüche, **dadurch gekennzeichnet, dass** das wenigstens eine kationische Tensid ausgewählt wird aus der Gruppe der Amidoamine, insbesondere aus der Gruppe Stearylamidopropyltrimmonium Chloride, Ricinoleamidopropyltrimonnium Chloride und/oder Palmitamidopropyltrimonium Chloride, weiter insbesondere ist das kationische Tensid Palmitamidopropyltrimonium Chlorid.

7. Emulsion nach wenigstens einem der vorstehenden Ansprüche, **dadurch gekennzeichnet, dass** das wenigstens eine kationische Tensid mit einem Gehalt von 0,1 bis 4 Gew.-%, bevorzugt 0.2 bis 2 Gew.-% enthalten ist, bezogen auf das Gesamtgewicht der Zubereitung und bezogen auf den Aktivgehalt.

8. Emulsion nach wenigstens einem der vorstehenden Ansprüche, **dadurch gekennzeichnet, dass** die wenigstens zwei Moisturizer ausgewählt werden aus der Gruppe Glycerin, Sorbitol, Butylenglykol, Propylenglykol, Milchsäure und/oder Lactate, insbesondere Natriumlactat, Biosaccaride Gum-1, Glycine Soja, Pyrrolidoncarbonsäure und Harnstoff, bevorzugt aus der Gruppe Glycerin, Sorbitol, Butylenglykol, Propylenglykol, Milchsäure und/oder Lactate, insbesondere Natriumlactate, besonders bevorzugt sind die zwei Moisturizer Glycerin und Sorbitol enthalten.

9. Emulsion nach wenigstens einem der vorstehenden Ansprüche, **dadurch gekennzeichnet, dass** die wenigstens zwei Moisturizer mit einem Gesamtgehalt von 5,5 bis 30,0 Gew.-%, bevorzugt 6,0 bis 27,0 Gew.-%, besonders bevorzugt 7,5 bis 25,0 Gew.-%, bezogen auf das Gesamtgewicht der Emulsion, vorliegen.

10. Emulsion nach wenigstens einem der vorstehenden Ansprüche, **dadurch gekennzeichnet, dass** der wenigstens eine Fettalkohol ausgewählt wird aus der Gruppe Myristylalkohol, Cetylalkohol, Stearylalkohol und/oder Cetearylalkohol, bevorzugt sind Myristylalkohol und/oder Cetylalkohol enthalten.

11. Emulsion nach wenigstens einem der vorstehenden Ansprüche, **dadurch gekennzeichnet, dass** der wenigstens eine Fettalkohol mit einem Gesamtgehalt von 4,0 bis 15,0 Gew.-%, bevorzugt von 4,5 bis 10,0 Gew.-% enthalten ist, bezogen auf das Gesamtgewicht der Emulsion.

12. Emulsion nach wenigstens einem der vorstehenden Ansprüche, **dadurch gekennzeichnet, dass** der wenigstens eine natürliche Verdicker ausgewählt wird aus der Gruppe der modifizierten Stärkeverbindungen, bevorzugt aus der Gruppe Reisstärke, Maisstärke oder Kartoffelstärke, besonders bevorzugt hydrolysierte Maisstärke.

13. Emulsion nach wenigstens einem der vorstehenden Ansprüche, **dadurch gekennzeichnet, dass** der wenigstens eine natürliche Verdicker mit einem Gesamtgehalt von 0,1 bis 8,0 Gew.-%, bevorzugt von 0,5 bis 5,0-Gew. % vorliegt, bezogen auf das Gesamtgewicht der Emulsion.

14. Produkt bestehend aus einer Haar-Styling-Emulsion gemäß wenigstens einem der vorstehenden Ansprüche und einer Verpackungseinheit, insbesondere ein Tiegel oder eine Tube.

15. Verwendung von Pullulan in einer Menge von 0,1 bis 10,0 Gew.-%, bezogen auf das Gesamtgewicht der Haar-Styling-Emulsion, in Kombination mit wenigstens zwei Moisturizern, wenigstens einem Fettalkohol, wenigstens einem Emulgator und/oder wenigstens einem kationischen Tensid, und optional wenigstens einem natürlichen Verdicker, in einer Haar-Styling-Emulsion zur Festigung der Haare und zum Frisurerhalt.

16. Verwendung nach Anspruch 15, **dadurch gekennzeichnet, dass** der wenigstens eine natürliche Verdicker hydrolysierte Maisstärke ist, bevorzugt vorliegend mit einem Gehalt von 0,1 bis 8,0 Gew.-%, bezogen auf das Gesamtgewicht der Haar-Styling-Emulsion.
